# FASCICULE DE BREVET EUROPEEN

(11) **EP 2 506 971 B1**
(45) Date de publication et mention de la délivrance du brevet: **18.04.2018**
(21) Numéro de dépôt: 10785386.3
(22) Date de dépôt: 18.11.2010
(51) Int. Cl.: C07C 45/53, C07C 29/132, C07C 29/00, B01J 31/02

(54) **PROCEDE DE PREPARATION D'UN CATALYSEUR DE DEPEROXYDATION**
VERFAHREN ZUR HERSTELLUNG EINES DEPEROXIDATIONSKATALYSATORS
METHOD FOR PREPARING A DEPEROXIDATION CATALYST

(30) Priorité: 30.11.2009 FR 0958500
(43) Date de publication de la demande: 10.10.2012
(73) Titulaire: Rhodia Operations, 93306 Aubervilliers (FR)
(72) Inventeur: CHOUZIER, Sandra, F-69006 Lyon (FR); VERACINI, Serge, F-69005 Lyon (FR); IGERSHEIM, Françoise, F-69003 Lyon (FR)
(74) Mandataire: Chatelan, Florence Anne
(86) Numéro de dépôt international: PCT/EP2010/067752
(87) Numéro de publication internationale: WO 2011/064135

(56) Documents cités:
- EP-A1- 0 023 464
- GB-A- 1 229 734
- US-A- 2 169 368
- US-A- 3 923 895
- US-A- 5 496 806

## Description

La présente invention concerne un procédé de préparation d'un catalyseur de déperoxydation comprenant comme élément catalytique principal du chrome.

Elle concerne plus particulièrement un procédé de préparation d'une solution organique d'un ester d'acide chromique, cette solution étant utilisée comme catalyseur dans l'étape de déperoxydation d'un peroxyde d'alkyle notamment dans l'étape de déperoxydation de l'hydroperoxyde de cyclohexyle, dans le procédé de fabrication de cyclohexanol/cyclohexanone par oxydation du cyclohexane. Le cyclohexanol et/ou cyclohexanone sont notamment des produits intermédiaires utilisés dans la fabrication d'acide adipique ou de l'epsilon-caprolactame, monomères pour la production de polyamides.

Certains procédés de synthèse de composés oxydés tels que des acides, diacides, alcools, cétones, utilisent comme matières premières ou composés intermédiaires des composés peroxydés. Ces composés doivent être déperoxydés ou décomposés dans une étape spécifique pour obtenir un ou plusieurs produits oxydés tels que des alcools, cétones, acides ou aldéhydes.

Un des grands procédés industriels appartenant à ce groupe, est le procédé de fabrication de diacides, plus particulièrement d'acide adipique, par oxydation de cyclohexane en hydroperoxyde de cyclohexyle puis décomposition par déperoxydation de ce peroxyde en alcool et/ou cétone. Ces derniers composés oxydés sont ensuite transformés en diacides, principalement acide adipique, par oxydation par l'acide nitrique.

L'oxydation du cyclohexane en hydroperoxyde de cyclohexyle est réalisée en milieu liquide par un gaz contenant de l'oxygène et en absence de catalyseur. Cette étape est notamment décrite dans les brevets GB 777087, 1112837, 964869, 1191573, US 3479394, US 4877903.

Après éventuellement élimination du cyclohexane qui n'a pas réagi et lavage à l'eau du milieu réactionnel pour récupérer et extraire certains sous-produits formés, l'hydroperoxyde de cyclohexyle est décomposé par déperoxydation en cyclohexanone et cyclohexanol, en présence d'un catalyseur comprenant comme élément catalytique principal du chrome, de préférence sous forme de composés de chrome à la valence 6+, tels que, par exemple un ester d'acide chromique. Cette étape est notamment décrite dans le brevet français 1580206.

EP-A-0023464 décrit un procédé de stabilisation au vieillissement des solutions chromiques, notamment les solutions cyclohexaniques de chromate de tertio-butyle par ajout d'un ester phosphorique.

Généralement, le catalyseur utilisé est en solution dans un milieu organique pour être présent dans le milieu réactionnel sous forme dissoute. Ainsi, le chromate de di-tertiobutyle est le composé de chrome préféré pour la décomposition de l'hydroperoxyde de cyclohexyle.
Un tel produit est connu et peut être synthétisé par différentes méthodes.
Toutefois, aucun procédé de fabrication décrit ne permet de fabriquer un tel catalyseur de manière productive, sous forme convenable pour être introduit dans un procédé de décomposition d'un hydroperoxyde d'alkyle et sans production d'effluents contenant du chrome, nocifs pour l'environnement.
A cet effet, l'invention propose un procédé de préparation d'un catalyseur de déperoxydation d'un hydroperoxyde d'alkyle comprenant comme élément catalytique principal du chrome à l'état d'oxydation 6+. Ce procédé comprend les étapes suivantes :
- Dissoudre dans de l'eau de l'anhydride chromique,
- Ajouter à la solution aqueuse d'anhydride chromique, un alcool tertiaire comprenant au moins 4 atomes de carbone et un solvant hydrocarbure,
- Faire réagir l'alcool et l'anhydride chromique pour obtenir un chromate d'alkyle, par mise sous pression réduite du milieu réactionnel à une température comprise entre 20 et 40 °C et distillation de l'eau contenue dans le milieu réactionnel,
- Récupérer le catalyseur sous forme d'ester de l'acide chromique en solution dans l'hydrocarbure.
Selon un mode préféré de réalisation de l'invention, la concentration d'anhydride chromique dans la solution aqueuse est comprise entre 35 et 75% en poids, de préférence entre 45 et 70% en poids et encore plus préférentiellement entre 55 et 65% en poids.
Le passage par l'acide chromique en solution aqueuse est avantageux pour des raisons de sécurité. En effet, la mise en oeuvre directe de l'anhydride chromique sur une solution organique comprenant un alcool tertiaire et un solvant hydrocarbure, n'est pas bien maîtrisable à l'échelle industrielle et présente le risque de générer un mélange instable, notamment en cas de défaut d'alimentation de l'alcool.

Selon une caractéristique de l'invention, l'eau est éliminée du milieu réactionnel par distillation sous forme d'azéotrope formé notamment avec le solvant hydrocarbure et/ou éventuellement l'alcool. L'élimination de l'eau à ce stade du procédé permet de s'affranchir de la formation de dépôts solides lors du stockage de la solution d'ester chromique.

Selon une autre caractéristique préférée de l'invention, la réaction chimique de l'alcool et de l'anhydride chromique en solution aqueuse et l'élimination de l'eau (eau formée au cours de la réaction et eau utilisée pour solubiliser l'anhydride chromique) sont opérées conjointement, en mode continu dans un même appareillage, avantageusement, une colonne réactive de distillation. Ainsi, la solution aqueuse d'anhydride chromique est alimentée dans la partie supérieure de la colonne, l'alcool et le solvant hydrocarbure étant introduits en tête de colonne. La solution de chromate d'alkyle est soutirée comme fraction de fond de la colonne. L'alcool et/ou l'hydrocarbure distillé conjointement avec l'eau est séparé, par exemple, par décantation et recyclé dans la colonne. Outre l'avantage de séparer toute phase aqueuse hétérogène de la solution d'ester chromique, évitant ainsi la formation de précipités de composés du chrome, l'élimination continue de l'eau du milieu réactionnel par distillation sous pression réduite permet une estérification quantitative de l'anhydride chromique engagé, et donc un procédé plus propre et plus productif.

Les solvants hydrocarbures convenables pour l'invention sont les hydrocarbures saturés linéaires ou cycliques tels que le cyclohexane, le cyclooctane, le cyclododécane, la décaline ou analogue.

De préférence, le solvant hydrocarbure est l'hydrocarbure correspondant à celui dont est dérivé le peroxyde à décomposer. Ainsi, dans le procédé de fabrication de cyclohexanol et/ou cyclohexanone, le composé à déperoxyder est l'hydroperoxyde de cyclohexyle obtenu par oxydation du cyclohexane par l'oxygène. Le solvant utilisé est avantageusement le cyclohexane.

Comme hydroperoxyde d'alkyle convenable pour l'invention, on peut citer les hydroperoxydes obtenus par oxydation par l'oxygène des hydrocarbures saturés cycliques comme le cyclohexane, le cyclooctane, le cyclododécane, ou la décaline.

Selon une autre caractéristique préférentielle de l'invention, les alcools convenables pour l'invention sont les alcools saturés tertiaires aliphatiques ramifiés ou non comprenant au moins 4 atomes de carbone. L'alcool tertiobutanol est l'alcool préféré.

Le procédé de l'invention permet une transformation pratiquement stoechiométrique de l'anhydride chromique en chromate d'alkyle. En effet, la réaction est mise en oeuvre dans des conditions de pression comprises entre 0,10 bar et 0,30 bar absolu et de température qui permettent d'éliminer l'eau contenue dans le milieu réactionnel et ainsi déplacer l'équilibre de la réaction d'estérification. De plus, le domaine de température compris entre 20 et 40°C, de préférence entre 25 et 35°C, évite la précipitation du chrome sous forme oxydée.

La concentration de chrome dans le milieu réactionnel et la quantité de solvant hydrocarbure ajoutée sont déterminées pour obtenir une solution d'ester chromique, de préférence de chromate de di-tertiobutyle, stable comprenant entre 2,5 % en masse et 8% en masse de chrome, de préférence entre 3 % en masse et 5% en masse, exprimée en élément chrome.

Avantageusement, la solution d'ester chromique est stabilisée par addition d'un stabilisant choisi parmi les esters de l'acide phosphorique, de préférence l'acide octylphosphorique ou l'acide isobutylphosphorique. L'addition du stabilisant peut être réalisée dans la solution obtenue ou par alimentation de stabilisant, notamment de l'acide octylphosphorique, dans la colonne réactive de distillation de l'eau décrite précédemment. De manière particulièrement avantageuse, le stabilisant est ajouté en quantité telle que le rapport molaire Phosphore/Chrome (P/Cr) soit inférieur à 0,05, de préférence compris entre 0,02 et 0,05. Le respect de ce rapport molaire P/Cr lors de l'addition du stabilisant, permet de prévenir la précipitation d'oxyde de chrome au cours du stockage des solutions d'ester chromique, sans désactiver le catalyseur.

Le procédé de fabrication de l'invention permet d'obtenir une solution d'ester chromique stable avec une très bonne productivité et un rendement élevé obtenu notamment par l'élimination en continu de l'eau. De plus, ce procédé produit un effluent, l'eau distillée, exempt de chrome.

La solution ainsi obtenue peut être utilisée directement comme catalyseur dans un procédé de déperoxydation, sans risque de précipitation de chrome car elle ne contient notamment pas d'eau.

Plus particulièrement, cette solution de chromate d'alkyle, de préférence de chromate de di-tertiobutyle est utilisée comme catalyseur dans l'étape de déperoxydation de l'hydroperoxyde de cyclohexyle pour produire un mélange de cyclohexanol/cyclohexanone par oxydation du cyclohexane. Ce mélange est oxydé avec l'acide nitrique pour synthétiser l'acide adipique.
En effet, dans ce procédé, le cyclohexane à l'état liquide est soumis à une oxydation par l'oxygène ou un gaz contenant de l'oxygène pour former l'hydroperoxyde de cyclohexyle et différents autres produits oxydés. Après séparation d'une partie du cyclohexane qui n'a pas réagi et éventuellement extraction par l'eau des composés oxydés tels que des hydroperoxydes aliphatiques primaires acides, des hydroxy acides et des diacides, l'hydroperoxyde de cyclohexyle en solution dans le cyclohexane est introduit dans une étape de déperoxydation pour être transformé en cyclohexanone et/ou cyclohexanol. Cette étape de déperoxydation est mise en oeuvre en présence d'un catalyseur à base de chrome à l'état d'oxydation 6+. Le catalyseur alimenté dans cette étape de déperoxydation est une solution de chromate d'alkyle, de préférence de chromate de di-tertiobutyle, obtenue selon un procédé de production conforme à la présente invention.
L'utilisation d'une solution organique de catalyseur préparée selon le procédé de l'invention permet de maîtriser la concentration en catalyseur actif (concentration en chrome à l'état d'oxydation 6+) dans le milieu réactionnel, et en conséquence le taux de conversion ou décomposition de l'hydroperoxyde de cyclohexyle. Le catalyseur reste sous forme dissoute dans le milieu réactionnel jusqu'à un taux de transformation très élevé de l'hydroperoxyde de cyclohexyle (taux de transformation supérieur à 90%). Dans le cas d'une utilisation d'une solution de catalyseur contenant de l'eau, il apparait un précipité d'oxyde de chrome qui est un inconvénient important pour la conduite du procédé et son économie.
D'autres avantages, détails de l'invention apparaitront au vu des exemples donnés ci-dessous uniquement à titre d'illustration, sans caractère limitatif.

### Exemple 1 : Préparation d'une solution de chromate de di-tertiobutyle selon l'invention

De l'anhydride chromique CrO₃ est dissout dans de l'eau pour obtenir une solution d'acide chromique à une concentration de 60% en poids.

Cette solution aqueuse d'acide chromique est alimentée avec un débit de 13 kg/h dans une colonne à distiller comprenant dix plateaux, au niveau d'un plateau situé à proximité de la tête de colonne.
L'alcool tertiobutanol et le solvant hydrocarbure (cyclohexane) sont alimentés en tête de colonne avec des débits respectifs de 16 et 90 kg/h.
De l'acide octylphosphorique est ajouté comme stabilisant en tête de colonne avec un débit de 1 kg/h.
Comme indiqué précédemment, ce composé peut être additionné dans la solution d'ester chromique récupérée en bas de colonne.
La colonne fonctionne sous une pression de 130 mm Hg (0,17 bar) avec une température de pied de colonne de 30°C.
En tête de la colonne à distiller, une fraction contenant l'azéotrope eau/tertiobutanol/cyclohexane est récupérée. Après décantation, la phase organique contenant le cyclohexane et de l'alcool est recyclée dans la colonne, la phase aqueuse, exempte de chrome, étant éliminée.
En pied de colonne à distiller, la solution de chromate de di-tertiobutyle est récupérée.
La solution ainsi préparée est alimentée dans le réacteur de décomposition de l'hydroperoxyde de cyclohexyle.

### Exemple 2 : Préparation d'une solution de chromate de di-tertiobutyle selon l'invention

De l'anhydride chromique CrO₃ est dissout dans de l'eau pour obtenir une solution d'acide chromique à une concentration de 60 % en poids.
Cette solution aqueuse d'acide chromique est alimentée avec un débit de 15 kg/h dans une colonne à distiller comprenant dix plateaux, au niveau d'un plateau situé à proximité de la tête de colonne.
L'alcool tertiobutanol et le solvant hydrocarbure (cyclohexane) sont alimentés en tête de colonne avec des débits respectifs de 21 et 125 kg/h.
De l'acide octylphosphorique est ajouté comme stabilisant en tête de colonne avec un débit de 1,3 kg/h.
La colonne fonctionne sous une pression de 120 mm Hg (0,16 bar) avec une température de pied de colonne de 29,5°C.
En tête de la colonne à distiller, une fraction contenant l'azéotrope eau/tertiobutanol/cyclohexane est récupérée. Après décantation, la phase organique contenant le cyclohexane et de l'alcool est recyclée dans la colonne, la phase aqueuse, exempte de chrome, étant éliminée.
En pied de colonne à distiller, la solution de chromate de di-tertiobutyle est récupérée.

La solution ainsi préparée est alimentée dans le réacteur de décomposition de l'hydroperoxyde de cyclohexyle.

### Exemple 3 : Préparation d'une solution de chromate de di-tertiobutyle selon l'invention

On reproduit l'exemple 2 ci-dessus, sauf que de l'acide isobutylphosphorique (au lieu d'acide octylphosphorique) est ajouté comme stabilisant en tête de colonne avec un débit de 0,75 kg/h.
La colonne fonctionne dans les mêmes conditions que celles décrites à l'exemple 2.
En tête de la colonne à distiller, une fraction contenant l'azéotrope eau/tertiobutanol/cyclohexane est récupérée. Après décantation, la phase organique contenant le cyclohexane et de l'alcool est recyclée dans la colonne, la phase aqueuse, exempte de chrome, étant éliminée.
En pied de colonne à distiller, la solution de chromate de di-tertiobutyle est récupérée.
La solution ainsi préparée est alimentée dans le réacteur de décomposition de l'hydroperoxyde de cyclohexyle.

### Exemple comparatif : Préparation d'une solution de chromate de di-tertiobutyle sans élimination d'eau en continu

De l'anhydride chromique CrO₃ est dissout dans de l'eau pour obtenir une solution d'acide chromique à une concentration de 70 % en poids.
Cette solution aqueuse d'acide chromique est alimentée sous agitation à 20°C (pendant 1heure), avec un débit de 85 kg/h, dans un réacteur batch comprenant 78 kg de tertiobutanol et 583 kg de cyclohexane.
Après la fin de la coulée, le mélange est maintenu encore 1heure sous agitation, puis laissé à décanter pour séparation de la couche aqueuse.
La quantité de Chrome présent dans la couche aqueuse est dosée. La couche aqueuse contient 17 % (en poids) du Chrome alimenté.

## Revendications

1. Procédé de préparation d'un catalyseur de déperoxydation d'un hydroperoxyde d'alkyle comprenant du chrome à l'état d'oxydation 6+ comme élément catalytique principal, **caractérisé en ce qu'**il comprend les étapes suivantes :
• Dissoudre dans de l'eau de l'anhydride chromique,
• Ajouter à la solution aqueuse d'anhydride chromique, un alcool tertiaire comprenant d'au moins 4 atomes de carbone et un solvant hydrocarbure,
• Faire réagir l'alcool et l'anhydride chromique par mise sous pression réduite du milieu pour obtenir une distillation de l'eau, à une température comprise entre 20 et 40°C,
• Récupérer le catalyseur sous forme d'ester de l'acide chromique en solution dans le solvant hydrocarbure.

2. Procédé selon la revendication 1, **caractérisé en ce que** l'eau est éliminée sous forme d'azéotrope formé avec le solvant hydrocarbure et/ou l'alcool.

3. Procédé selon l'une des revendications 1 ou 2 **caractérisé en ce que** la réaction de l'alcool avec l'anhydride chromique en solution aqueuse et l'élimination de l'eau sont réalisées conjointement, en mode continu dans un même appareillage.

4. Procédé selon la revendication 3, **caractérisé en ce que** l'appareillage est une colonne réactive de distillation, la solution aqueuse d'anhydride chromique étant alimentée dans la partie supérieure de la dite colonne, l'alcool et l'hydrocarbure étant alimentés en tête de colonne, la solution d'ester de l'acide chromique étant soutirée comme fraction de fond de colonne.

5. Procédé selon l'une des revendications précédentes, **caractérisé en ce que** le solvant hydrocarbure est choisi dans le groupe comprenant les hydrocarbures saturés.

6. Procédé selon la revendication 5, **caractérisé en ce que** l'hydrocarbure est choisi dans le groupe comprenant le cyclohexane, le cyclooctane, le cyclododécane et la décaline.

7. Procédé selon l'une des revendications précédentes, **caractérisé en ce que** l'alcool est le tertiobutanol.

8. Procédé selon l'une des revendications précédentes, **caractérisé en ce que** la pression à laquelle est réalisée l'étape d'estérification est comprise entre 10 kPa et 30 kPa absolu.

9. Procédé selon l'une des revendications précédentes, **caractérisé en ce que** la solution organique d'ester chromique contient entre 2,5% et 8% en masse de chrome exprimé en masse d'élément chrome.

10. Procédé selon l'une des revendications précédentes, **caractérisé en ce que** la solution organique d'ester chromique est stabilisée par addition d'un stabilisant choisi parmi les esters de l'acide phosphorique, de préférence le stabilisant est l'acide octylphosphorique ou l'acide isobutylphosphorique.

11. Procédé selon la revendication 10, **caractérisé en ce que** le stabilisant est ajouté en quantité telle que le rapport molaire P/Cr soit inférieur à 0,05.

12. Procédé selon la revendication 10 ou 11, **caractérisé en ce que** le stabilisant est alimenté en tête de colonne réactive de distillation.

## Patentansprüche

1. Verfahren zur Herstellung eines Katalysators für die Deperoxidierung eines Alkylhydroperoxids, der Chrom im Oxidationszustand 6+ als hauptsächliches katalytisches Element umfasst, **dadurch gekennzeichnet, dass** es die folgenden Schritte umfasst:
• Lösen von Chromsäureanhydrid in Wasser,
• Zugeben zu der wässrigen Chromsäureanhydridlösung eines tertiären Alkohols, der mindestens 4 Kohlenstoffatome umfasst, und eines Kohlenwasserstofflösungsmittels,
• Umsetzen des Alkohols und des Chromsäureanhydrids, indem das Medium unter verringertem Druck gebracht wird, um eine Destillation des Wassers zu erhalten, bei einer Temperatur zwischen 20°C bis 40°C,
• Rückgewinnen des Katalysators in Form von Chromsäureester in Lösung in dem Kohlenwasserstofflösungsmittel.

2. Verfahren nach Anspruch 1, **dadurch gekennzeichnet, dass** das Wasser in Form eines mit dem Kohlenwasserstofflösungsmittel und/oder dem Alkohol gebildeten Azeotrops entfernt wird.

3. Verfahren nach einem der Ansprüche 1 oder 2, **dadurch gekennzeichnet, dass** die Umsetzung des Alkohols mit dem Chromsäureanhydrid in wässriger Lösung und die Entfernung des Wassers gleichzeitig im kontinuierlichen Modus in derselben Vorrichtung durchgeführt werden.

4. Verfahren nach Anspruch 3, **dadurch gekennzeichnet, dass** es sich bei der Vorrichtung um eine Reaktivdestillationskolonne handelt, wobei die wässrige Chromsäureanhydridlösung im oberen Teil der Kolonne zugeführt wird, der Alkohol und der Kohlenwasserstoff am Kopf der Kolonne zugeführt werden, wobei die Chromsäureesterlösung als Sumpffraktion der Kolonne entnommen wird.

5. Verfahren nach einem der vorhergehenden Ansprüche, **dadurch gekennzeichnet, dass** das Kohlenwasserstofflösungsmittel aus der gesättigte Kohlenwasserstoffe umfassenden Gruppe ausgewählt ist.

6. Verfahren nach Anspruch 5, **dadurch gekennzeichnet, dass** der Kohlenwasserstoff aus der Gruppe ausgewählt ist, die Cyclohexan, Cyclooctan, Cyclododecan und Decalin umfasst.

7. Verfahren nach einem der vorhergehenden Ansprüche, **dadurch gekennzeichnet, dass** es sich bei dem Alkohol um tert-Butanol handelt.

8. Verfahren nach einem der vorhergehenden Ansprüche, **dadurch gekennzeichnet, dass** der Druck, bei dem der Veresterungsschritt durchgeführt wird, zwischen 10 kPa und 30 kPa absolut beträgt.

9. Verfahren nach einem der vorhergehenden Ansprüche, **dadurch gekennzeichnet, dass** die organische Chromsäureesterlösung zwischen 2,5 und 8 Massen-% Chrom, ausgedrückt als Masse an elementarem Chrom, enthält.

10. Verfahren nach einem der vorhergehenden Ansprüche, **dadurch gekennzeichnet, dass** die organische Chromsäureesterlösung durch Zugabe eines Stabilisators, ausgewählt aus Phosphorsäureestern, stabilisiert wird, wobei der Stabilisator vorzugsweise Octylphosphorsäure oder Isobutylphosphorsäure ist.

11. Verfahren nach Anspruch 10, **dadurch gekennzeichnet, dass** der Stabilisator in einer derartigen Menge zugegeben wird, dass das Molverhältnis P/Cr kleiner als 0,05 ist.

12. Verfahren nach Anspruch 10 oder 11, **dadurch gekennzeichnet, dass** der Stabilisator am Kopf der Reaktivdestillationskolonne zugeführt wird.

## Claims

1. Process for preparing a catalyst for the deperoxidation of an alkyl hydroperoxide comprising chromium in the 6+ oxidation state as the main catalytic element, **characterized in that** it comprises the following steps:
• dissolving chromic anhydride in water,
• adding to the aqueous solution of chromic anhydride, a tertiary alcohol comprising at least 4 carbon atoms and a hydrocarbon solvent,
• reacting the alcohol and the chromic anhydride by putting the medium under reduced pressure in order to distil the water, at a temperature between 20 and 40°C,
• recovering the catalyst in the form of a chromic acid ester in solution in the hydrocarbon solvent.

2. Process according to Claim 1, **characterized in that** the water is removed in the form of an azeotrope formed with the hydrocarbon solvent and/or the alcohol.

3. Process according to either of Claims 1 or 2, **characterized in that** the reaction of the alcohol with the chromic anhydride in aqueous solution and the removal of the water are carried out at the same time, in continuous mode in one and the same apparatus.

4. Process according to Claim 3, **characterized in that** the apparatus is a reactive distillation column, the aqueous solution of chromic anhydride being fed into the upper part of said column, the alcohol and the hydrocarbon being fed into the top of the column, the solution of chromic acid ester being drawn off as a fraction from the bottom of the column.

5. Process according to one of the preceding claims, **characterized in that** the hydrocarbon solvent is selected from the group consisting of saturated hydrocarbons.

6. Process according to Claim 5, **characterized in that** the hydrocarbon is selected from the group consisting of cyclohexane, cyclooctane, cyclododecane and decalin.

7. Process according to one of the preceding claims, **characterized in that** the alcohol is *tert*-butanol.

8. Process according to one of the preceding claims, **characterized in that** the pressure at which the esterification step is carried out is between 10 kPa and 30 kPa absolute.

9. Process according to one of the preceding claims, **characterized in that** the organic chromic ester solution contains between 2.5% and 8% by weight of chromium expressed as weight of elemental chromium.

10. Process according to one of the preceding claims, **characterized in that** the organic chromic ester solution is stabilized by addition of a stabilizer chosen from esters of phosphoric acid, preferably the stabilizer is octylphosphoric acid or isobutylphosphoric acid.

11. Process according to Claim 10, **characterized in that** the stabilizer is added in an amount such that the P/Cr molar ratio is less than 0.05.

12. Process according to Claim 10 or 11, **characterized in that** the stabilizer is fed into the top of the reactive distillation column.
